## Europäisches Patentamt

## European Patent Office

## Office européen des brevets

(19)

(11) Veröffentlichungsnummer: **0 151 241**
**B1**

(12) **EUROPÄISCHE PATENTSCHRIFT**

(45) Veröffentlichungstag der Patentschrift:
07.10.87

(21) Anmeldenummer: **84113804.3**

(22) Anmeldetag: **15.11.84**

(51) Int. Cl.⁴: **C 07 C 47/12,** C 07 C 45/51,
C 07 C 45/49, C 07 C 47/198

(54) **Verfahren zur Herstellung von 1,4 Butandial.**

(30) Priorität: **01.02.84 DE 3403427**

(43) Veröffentlichungstag der Anmeldung:
**14.08.85 Patentblatt 85/33**

(45) Bekanntmachung des Hinweises auf die Patenterteilung:
**07.10.87 Patentblatt 87/41**

(84) Benannte Vertragsstaaten:
**AT BE CH DE FR GB IT LI NL SE**

(56) Entgegenhaltungen:
**EP - A - 0 028 892**
**FR - A - 2 535 711**
**GB - A - 1 155 166**
**US - A - 2 626 283**

**J. ORG. CHEM. Band 37, Nr. 11, 1972, Seiten 1835-1837, C. BOTTEGHI et al.: "A convenient synthetic approach to 3- and 4-alkyl-2,3-dihydrofurans".**

(73) Patentinhaber: **Degussa Aktiengesellschaft, Weissfrauenstrasse 9, D-6000 Frankfurt am Main 1 (DE)**

(72) Erfinder: **Andrade, Juan, Dr. Dipl.-Chem., Hungerweg 5, D-8752 Kleinostheim (DE)**
Erfinder: **Prescher, Günter, Dr. Dipl.-Chem., Liesingstrasse 2, D-6450 Hanau 9 (DE)**
Erfinder: **Samson, Marc, Dr. Dipl.-Chem., A. Vermeylenstraat 2, B-9100 Lokeren (BE)**

## Beschreibung

Die Erfindung betrifft ein Verfahren zur Herstellung von 1,4-Butandial (Succindialdehyd) aus Acrolein unter Überführung des Acroleins in ein Acetal, Hydroformylierung des Acetals und Hydrolyse des Hydroformylierungsprodukts.

Es ist bekannt, 1,4-Butandial herzustellen, indem Tetrahydrofuran chloriert wird und das hierbei entstehende 2,5-Dichlortetrahydrofuran oder die aus diesem gebildeten 2,5-Dialkoxytetrahydrofurane zum 1,4-Butandial hydrolysiert werden (DD-PS 25656). Nachteilig ist bei diesem Verfahren, dass die Ausbeute, insbesondere bei der Chlorierung, schlecht ist.

Es ist auch bekannt, vom Acrolein zum 1,4-Butandial zu gelangen. Hierzu wird aus dem Acrolein zunächst Allylidendiacylat gebildet, dieses zum 4,4-Diacyloxybutyraldehyd hydroformyliert, aus diesem durch Umsetzung mit einem Alkanol 2,5-Dialkoxytetrahydrofuran gebildet und schliesslich dieses zum 1,4-Butandiol hydrolysiert (US-PS 2 920 081). Auch dieses Verfahren ergibt nur mässige Ausbeuten und ist zudem umständlich und daher für eine Anwendung in technischem Masstab wenig geeignet.

Es ist weiterhin bekannt, aus dem Acrolein zunächst das Acetal zu bilden, dieses in Gegenwart eines Kobaltkatalysators bei 120 bis 200°C und 50 bis 300 bar zu hydroformylieren und das Hydroformylierungsprodukt zu hydrolysieren (GB-PS 702 206). Während hierbei die Acetale des Acroleins mit niederen einwertigen Alkanolen, dass heisst acyclische Acetale, zu schlechten Ausbeuten führen, verläuft die Hydroformylierung bei Acetalen des Acroleins mit mehrwertigen Alkanolen, das heisst bei cyclischen Acetalen, erheblich günstiger, die Ausbeute an 1,4-Butandial ist dennoch unbefriedigend.

Aus der EP-Al-0 094 748 ist ein Katalysator, bestehend aus Rh H(CO) (PPh₃), und seine Verwendung für die Herstellung von gesättigten Dialdehyden durch Hydroformylierung ungesättigter Kohlenwasserstoffverbindungen mit 4 bis 6 Kohlenstoffatomen bekannt.

Führt man die Reaktion in Abwesenheit von Säuren durch, sind erhebliche Mengen an polymeren Nebenprodukten zu erwarten.

Aus der Ep-Al-0 028 892 ist die Hydroformylierung Acroleindiäthylacetal (d.h. 3,3-Diäthoxy-1-propen) zur Herstellung der entsprechenden Aldehyde (d.h. 4,4-Diäthoxybutanal und 2-Methyl-3,3-diäthoxypropanal) bekannt. Das Verfahren wird in Gegenwart eines Katalysators, der aus Carbonytriphenylphosphin hergestellt worden ist, durchgeführt.

Die genannten Isomeren werden jedoch in einem Molverhältnis von 1,5:1 gebildet.

Es ist nun ein Verfahren zur Herstellung von 1,4-Butandial aus Acrolein unter Überführung des Acroleins in ein Acetal, Hydroformylierung des Acetals und Hydrolyse des Hydroformylierungsprodukts gefunden worden, das dadurch gekennzeichnet ist, dass man

a. das Acrolein in ein 3,3-Dialkoxy-1-propen überführt,

b. dieses in Gegenwart von Hydridotris-triphenylphosphin-rhodiumcarbonyl sowie Triphenylphosphin und beziehungsweise oder Triphenylphosphit als Katalysator hydroformyliert,

c. aus Hydroformylierungsprodukt das 4,4-Dialkoxybutanal durch Destillation abtrennt und

d. dieses zum 1,4-Butandial hydrolysiert.

Dieses Verfahren ergibt wesentlich günstigere Ausbeuten an 1,4-Butandial als die bekannten Verfahren. Hierfür ist insbesondere die Stufe der Hydroformylierung massgebend. Während bisher die Hydroformylierung acyclischer Acetale nicht in brauchbarer Weise durchführbar ist und infolgedessen die Herstellung von 1,4-Butandial auf diesem Wege nicht in Frage kommt, führt die erfindungsgemässe Hydroformylierung dieser Acetale zu so hervorragenden Ergebnissen, dass nunmehr die Herstellung von 1,4-Butandial auf diesem Wege besonders vorteilhaft ist.

Zur Durchführung des erfindungsgemässen Verfahrens wird zunächst das Acrolein mit einem Alkanol zu einem 3,3-Dialkoxy-1-propen umgesetzt. Vorzugsweise wird ein 3,3-Dialkoxy-1-propen gewählt, das in jeder Alkoxy-Gruppe 1 bis 6 Kohlenstoffatome aufweist. Besonders geeignet sind das 3,3-Dimethoxy-1-propen und das 3,3-Diäthoxy-1-propen. Die Umsetzung erfolgt in bekannter Weise, zum Beispiel nach dem in Org. Synth. Coll. Vol. 4 (1963), Seite 21 bis 22, beschriebenen Verfahren durch Einwirkung der Orthoameisensäureester der entsprechenden Alkanole auf Acrolein oder nach dem in der DE-PS 930 752 oder der US-PS 2 626 283 beschriebenen Verfahren durch Einwirkung bei betreffenden Alkanole auf Acrolein in Gegenwart von starken Säuren als Katalysatoren.

Mit Vorteil wird für die Herstellung des 3,3-Dimethoxy-1-propens und des 3,3-Diäthoxy-1-propens das Verfahren gemäss gleichzeitiger Patentanmeldung P 3 403 426 angewendet. Nach diesem Verfahren wird das Acrolein in Gegenwart fester saurer Katalysatoren, wie stark saurer Ionenaustauscher oder stark saurer Zeolithe, mit dem Alkanol umgesetzt und das 3,3-Dialkoxy-1-propen gewonnen, indem das Umsetzungsgemisch nach der Abtrennung der Katalysatoren mittels inerten mit Wasser nicht mischbaren organischen Lösungsmitteln extrahiert und der Extrakt fraktionierend destilliert wird.

Zur weiteren Durchführung des erfindungsgemässen Verfahrens wird das 3,3-Dialkoxy-1-propen durch Umsetzung mit einem Gasgemisch aus Wasserstoff und Kohlenmonoxid in Gegenwart eines Katalysators hydroformyliert. Die Umsetzung erfolgt zweckmässigerweise bei erhöhter Temperatur, vorzugsweise bei Temperaturen etwa zwischen 100 und 140°C. Der Druck kann weitgehend beliebig gewählt werden, jedoch ist es im allgemeinen zweckmässig, wenigstens bei Normaldruck zu arbeiten. Bevorzugt werden Drücke etwa zwischen 1 und 6 bar. Zweckmässig ist es, mindestens stöchiometrische Mengen, vorzugsweise überschüssige Mengen, Wasserstoff und Kohlenmonoxid anzuwenden, wobei das Molverhältnis Wasserstoff zu Kohlenmonoxid weitgehend beliebig gewählt werden kann, vorzugsweise jedoch zwischen 0,5 und 1,0 bis 1,0 zu 0,5 liegen.

3 0 151 241 4

Bei der Hydroformylierung dient als Katalysator Hydridotris-triphenylphosphin-rhodiumcarbonyl im Gemisch mit Triphenylphosphin und beziehungsweise oder Triphenylphosphit. Derartige Katalysatoren sind in der DE-AS 1 793 069 beschrieben. Vorzugsweise werden bei der Durchführung des erfindungsgemässen Verfahrens je Gewichtsteil des 3,3-Dialkoxy-1-propens etwa 0,0001 bis 0,0025 Gewichtsteile des Hydridotristriphenylphosphinrhodiumcarbonyls und 0,04 bis 0,06 Gewichtsteile des Triphenylphosphins oder Triphenylphosphits angewendet.

Das Hydroformylierungsprodukt wird zwecks Gewinnung des 4,4-Dialkoxybutanals fraktionierend destilliert. Hierbei wird zweckmässigerweise unter vermindertem Druck gearbeitet, vorzugsweise bei Drücken unter 50 mbar.

Das so gewonnene 4,4-Dialkoxybutanal wird schliesslich durch Hydrolyse in das 1,4-Butandial übergeführt. Die hydrolyse erfolgt in saurem Medium, vorzugsweise in stark saurem Medium, mit besonderem Vorteil in Gegenwart eines sauren Ionenaustauschers, zweckmässigerweise bei Temperaturen unter 30°C, vorzugsweise bei Temperaturen zwischen 5 und 15°C.

*Beispiele*

1. In eine Mischung aus 146 g (2,6 mol) Acrolein und 480 g (3,2 mol) Orthoameisensäureäthylester wurde eine warme Lösung von 12 g Ammoniumnitrat in 170 ml wasserfreiem Äthanol eingetragen. Die Mischung wurde 6 Stunden stehengelassen, dann filtriert, mit 13 g Natriumcarbonat versetzt und fraktionierend destilliert. Die Fraktion, die bei 120 bis 125°C überging, enthielt das gebildete 3,3-Diäthoxy-1-propen. Die Ausbeute betrug 260 g (2,0 mol), entsprechend 77%, bezogen auf das eingesetzte Acrolein. Das 3,3-Diäthoxy-1-propen wurde in einem Rührautoklaven mit 9,4 g Triphenylphosphit und 0,4 g Hydridotris-triphenylphosphin-rhodiumcarbonyl vorgelegt. Dann wurde unter 3 bar Druck eine Mischung aus gleichen Volumenteilen Wasserstoff und Kohlenmonoxid eingespeist. Die Temperatur im Autoklaven wurde hierbei auf 110°C gehalten. Nach 140 Minuten wurde kein Gas mehr aufgenommen und die Einspeisung beendet. Durch gaschromatographische Untersuchung wurde festgestellt, dass 99,5% des 3,3-Diäthoxy-1-propens umgesetzt waren. Das Umsetzungsgemisch enthielt 4,4-Diätoxy-butanal und 2-Methyl-3,3-diäthoxy-propanal im Molverhältnis 8,5 zu 1,0. Das Umsetzungsgemisch wurde bei 20 mbar destilliert. Das gewünschte 4,4-Diäthoxy-butanal ging bei 90 bis 91°C über. Es wurde in gleichen Volumenteilen Wasser gelöst, und die Lösung wurde mit 60 g Ionenaustauscherharz DOWEX MSC-1 versetzt. Die Mischung wurde 2 Stunden lang bei 20°C gerührt und dann filtriert. Das Filtrat war eine 25%ige wässrige Lösung des 1,4-Butandials. Die Ausbeute an 1,4--Butandial, bezogen auf das eingesetzte 3,3-Diäthoxy-1-propen, betrug 80%.

2. Es wurde wie nach Beispiel 1 verfahren, jedoch wurden 208 g (1,6 mol) des 3,3-Diäthoxy-1-propens mit 7,5 g Triphenylphosphin und 0,4 g Hydridotris--triphenylphosphin-rhodiumcarbonyl vorgelegt, und die Hydroformylierung erfolgte bei 5 bar. 99,5% des 3,3-Diäthoxy-1-propens wurden umgesetzt. Das Umsetzungsgemisch enthielt 4,4-diäthoxy-butanal und 2-Methyl-3,3-diäthoxy-propanal im Molverhältnis 5,1 zu 1,0. Die Ausbeute an 1,4-Butandial, bezogen auf das eingesetzte 3,3-Diäthoxy-1-propen, betrug 71%.

3. Es wurde wie nach Beispiel 1 verfahren, jedoch wurden 195 g (1,5 Mol) des 3,3-Diäthoxy-1-propens mit 9,4 g Triphenylphosphit und 0,4 g Hydridotris--triphenylphosphin-rhodiumcarbonyl vorgelegt. Die Umsetzung erfolgte bei 140°C; die Gasaufnahme war nach 200 Minuten beendet. 99,4% des 3,3-Diäthoxy-1-propens waren umgesetzt. Das Umsetzungsgemisch enthielt das 4,4-Diäthoxy-butanal und 2-methyl-3,3-diäthoxy-propanal im Molverhältnis 11,2 zu 1,0. Die Ausbeute an 1,4-Butandial, bezogen auf das eingesetzte 3,3-Diäthoxy-1-propen, betrug 86%.

4. Es wurden stündlich 169,3 g (3,02 mol) Acrolein mit 189,1 g (5,91 mol) Methanol vermischt in gleichmässigem Strom in einen Schlaufenreaktor eingespeist. Diesem Strom wurden laufend die aus dem Umsetzungsgemisch zurückgewonnenen nicht umgesetzten Anteile Acrolein und Methanol zugeführt. In dem Kreislauf des Reaktors durchströmte die Mischung eine mit 100 g stark saurem Ionenaustauscherharz (Dowex MSC-1) gefüllte Zone. Die Temperatur in dem Reaktor wurde auf 17°C gehalten, die mittlere Verweilzeit war 2,3 Stunden. Das Umsetzungsgemisch wurde in gleichmässigem Strom aus dem Reaktor abgezogen und in die Mitte einer pulsierenden Extraktionskolonne geleitet. In die Kolonne wurden stündlich von oben 1810 g Wasser und von unten 810 g n-Octan eingespeist. Die Temperatur in der Kolonne war 20°C. Die aus der Kolonne oben und unter abgezogenen Phasen wurden fraktionierend destilliert. Hierbei fielen stündlich aus der oberen Phase 290,0 g (2,84 mol) 3,3-Dimethoxy--1-propen an. Ausserdem wurden stündlich aus der oberen Phase 6,0 g Acrolein und aus der unteren Phase 111,1 g Acrolein und 450,9 g Methanol zurückgewonnen. Es waren folglich 59,1% der insgesamt stündlich eingesetzten 286,4 g Acrolein umgesetzt. Das zurückgewonnene Acrolein und Methanol wurden laufend in den Schlaufenreaktor zurückgeführt. Das 3,3-Dimethoxy-1-propen war 98%ig. Sein Siedepunkt war 89 bis 90°C. Die Ausbeute, bezogen auf das umgesetzte Acrolein, betrug 94,0%. Im übrigen wurde weiter wie nach Beispiel 1 verfahren, jedoch wurden 204,3 g (2,0 mol) des gewonnenen 3,3-Dimethoxy-1-propens im Rührautoklaven vorgelegt. 99,5% des 3,3-Dimethoxy-1-propens wurden umgesetzt. Das Umsetzungsgemisch enthielt 4,4-Dimethoxy-butanal und 2-Methyl-3,3-dimethoxy-propanal im Verhältnis 10,3 zu 1,0. Das Umsetzungsgemisch wurde bei 18 mbar destilliert. Das gewünschte 4,4-Dimethoxy-butanal ging bei 69 bis 70°C über. Es wurde weiter wie nach Beispiel 1 behandelt. Die Ausbeute an 1,4-Butandial, bezogen auf das eingesetzte 3,3-Dimethoxy-1-propen, betrug 79%.

3

## Patentansprüche

1. Verfahren zur Herstellung von 1,4-Butandial aus Acrolein unter Überführung des Acroleins in ein Acetal, Hydroformylierung des Acetals und Hydrolyse des Hydroformylierungsprodukts dadurch gekennzeichnet, dass man

a. das Acrolein in ein 3,3-Dialkoxy-1-propen überführt,

b. dieses in Gegenwart von Hydridotris-triphenylphosphin-rhodiumcarbonyl sowie Triphenylphosphin und beziehungsweise oder Triphenylphosphit als Katalysator hydroformyliert,

c. aus dem Hydroformylierungsprodukt das 4,4--Dialkoxybutanal durch Destillation abtrennt und

d. dieses zum 1,4-Butandial hydrolysiert.

2. Verfahren nach Anspruch 1, dadurch gekennzeichnet, dass man je Gewichtsteil des 3,3-Dialkoxy--1-propens etwa 0,0001 bis 0,0025 Gewichtsteile des Hydridotris-triphenylphosphin-rhodiumcarbonyls und 0,04 bis 0,06 Gewichtsteile des Triphenylphosphins oder Triphenylphosphits anwendet.

3. Verfahren nach Anspruch 1 oder 2, dadurch gekennzeichnet, dass man die Hydroformylierung bei Temperaturen von 100 bis 140°C und Drücken von 1 bis 6 bar durchführt.

## Claims

1. A process for the production of 1,4-butane dialdehyde from acrolein with conversion of the acrolein into an acetal, hydroformylation of the acetal and hydrolysis of the hydroformylation product, characterised in that

a) the acrolein is converted into a 3,3-dialkoxy-1--propene,

b) the 3,3-dialkoxy-1-propene is hydroformylated in the presence of hydridotris-triphenylphosphene rhodiumcarbonyl and triphenylphosphine and/or triphenylphosphite as a catalyst,

c) the 4,4-dialkoxy butane aldehyde is separated from the hydroformylation product by distillation and

d) the 4,4-dialkoxy butane aldehyde is hydrolysed to 1,4-butane dialdehyde.

2. A process according to claim 1, characterised in that about 0.0001 to 0.0025 parts by weight of the hydridotris-triphenylphosphine rhodiumcarbonyl and from 0.04 to 0.06 parts by weight of the triphenylphosphine or triphenylphosphite is used for each part by weight of 3,3-dialkoxy-1--propene.

3. A process according to claim 1 or 2, characterised in that hydroformylation is carried out at temperatures of from 100 to 140°C and at pressures of from 1 to 6 bar.

## Revendications

1. Procédé d'obtention de 1,4-butane-dialdéhyde à partir d'acroléine par transformation de l'acroléine en un acétal, hydroformylation de l'acétal et hydrolyse du produit d'hydroformylation caractérisé en ce que:

a) l'acroléine est transformée en 3,3-dialcoxypropène-1,

b) on hydroformyle ce dernier en présence d'hydridotris-triphénylphosphine-rhodium-carboxyle, ainsi que de triphénylphosphine et/ou de phosphate-triphényl comme catalyseur,

c) on sépare à partir du produit d'hydroformylation le 4,4-dialcoxybutanal par distillation,

d) et hydrolyse celui-ci en 1,4-butanedialdéhyde.

2. procédé selon le revendication 1, caractérisé en ce que pour chaque partie en poids de 3,3-dialcoxypropène on utilisait environ 0,0001 à 0,0025 partie en poids d'hydridotris-phényl--phosphine-rhodium-carbonyle et de 0,04 à 0,06 parties en poids de triphénylphosphine ou de phosphite de triphényl.

3. Procédé selon la revendication 1 ou la revendication 2, caractérisé en ce que l'hydroformylation est effectuée à des températures allant de 100 à 140°C et des pressions de 1 à 6 bars.